# EUROPÄISCHE PATENTSCHRIFT

(11) **EP 3 332 736 B1**
(45) Veröffentlichungstag und Bekanntmachung des Hinweises auf die Patenterteilung: **04.11.2020**
(21) Anmeldenummer: 16203381.5
(22) Anmeldetag: 12.12.2016
(51) Int. Cl.: A61C 9/00, A61B 5/055

(54) **VERFAHREN ZUR ERFASSUNG EINES DENTALEN OBJEKTS**
METHOD FOR DETECTING A DENTAL OBJECT
PROCÉDÉ DE DÉTECTION D'UN OBJET DENTAIRE

(43) Veröffentlichungstag der Anmeldung: 13.06.2018
(73) Patentinhaber: Sirona Dental Systems GmbH, 64625 Bensheim (DE)
(72) Erfinder: Braun, Tim, 64521 Groß-Gerau (DE); Abkai, Ciamak, 68542 Heddesheim (DE); Paul, Jan, 89073 Ulm (DE); Rasche, Volker, 89155 Erbach (DE)
(74) Vertreter: Özer, Alpdeniz

(56) Entgegenhaltungen:
- JP-A- 2003 113 096
- US-A1- 2009 128 553
- US-A1- 2013 252 196
- US-A1- 2015 110 245

## Beschreibung

### Technisches Gebiet

Die Erfindung betrifft ein Verfahren zur Erfassung eines dentalen Objekts mit einem Objektvolumen, insbesondere zumindest eines Teils eines Oberkiefers und/oder eines Unterkiefers, mittels einer MRT-Vorrichtung, wobei mehrere MRT-Segmentaufnahmen mittels der MRT-Vorrichtung innerhalb eines Messvolumens der MRT-Vorrichtung aufgenommen werden, die festgelegte Segment-Volumenbereiche abbilden, wobei die Segment-Volumenbereiche sich höchstens teilweise überlappen.

### Stand der Technik

Aus dem Stand der Technik sind mehrere Verfahren zur Erstellung von MRT-Aufnahmen von dentalen Objekten oder Teilen davon, wie von Ober- / Unterkiefern, bekannt. US2013/0252196 offenbart ein Verfahren und eine MRT-Vorrichtung zur Vermessung eines Patientenkopfes.

Nach einem bekannten Verfahren werden mittels einer herkömmlichen MRT-Vorrichtung einzelne MRT-Schnittaufnahmen durchgeführt, die beispielsweise den Kieferbogen schneiden. Zur Aufnahme des gesamten Kieferbogens sind dann beispielsweise fünf einzelne MRT-Schnittaufnahmen notwendig.

Ein Nachteil dieses Verfahrens besteht darin, dass die manuelle Planung der einzelnen MRT-Schnittaufnahmen mit erheblichem Zeitaufwand verbunden ist. Die einzelnen MRT-Schnittaufnahmen sind nicht zusammenhängend und erschweren damit die Diagnose.

Nach einem weiteren Verfahren dem sogenannten Curved-MPR-Verfahren wird zunächst ein annähernd isotroper Volumendatensatz mittels einer herkömmlichen MRT-Vorrichtung aufgezeichnet, der dann mittels eines Computers entlang eines manuell eingezeichneten gekrümmten Pfades über den Kieferbogen, einer sogenannten Panoramakurve, projiziert wird und dadurch eine zweidimensionale Produktionsaufnahme des gesamten Kieferbogens erzeugt wird.

Ein Nachteil dieses Verfahrens besteht darin, dass die Vermessung eines isotropen Volumendatensatzes im Vergleich zu einzelnen MRT-Schnittaufnahmen mit erheblich längerer Aufnahmedauer verbunden ist. Darüber hinaus wird die Bildqualität des Volumendatensatzes durch die nachträgliche Bildmanipulation, insbesondere durch einen Interpolationsverlust, verschlechtert.

Die Aufgabe der vorliegenden Erfindung besteht daher darin ein Verfahren zur Erfassung eines dentalen Objekts bereitzustellen, bei dem die Aufnahmedauer verkürzt wird und die Bildqualität verbessert wird, ohne gegenüber einer herkömmlichen MRT-Vorrichtung zusätzliche Hardwarekomponenten zu benötigen.

### Darstellung der Erfindung

Die Erfindung betrifft ein Verfahren zur Erfassung eines dentalen Objekts mit einem Objektvolumen, insbesondere zumindest eines Teils eines Oberkiefers und/oder eines Unterkiefers, mittels einer MRT-Vorrichtung, wobei mehrere MRT-Segmentaufnahmen mittels der MRT-Vorrichtung innerhalb eines Messvolumens der MRT-Vorrichtung aufgenommen werden, die jeweils festgelegte Segment-Volumenbereiche abbilden. , wie in Anspruch 1 definiert. Die Segment-Volumenbereiche überlappen sich dabei höchstens teilweise. Eine Zielfläche wird festgelegt oder ist bereits festgelegt. Computergestützt wird aus den einzelnen MRT-Segmentaufnahmen ein zweidimensionales Kompositbild erzeugt, das einem zweidimensionalen Aggregationsbild durch die Zielfläche innerhalb des Objektvolumens des dentalen Objekts entspricht.

Das dentale Objekt kann zumindest ein Teil des Oberkiefers und/oder des Unterkiefers sein. Das dentale Objekt kann auch mehrere nicht zusammenhängende Bereiche des Oberkiefers und/oder des Unterkiefers beinhalten. Die Kiefergelenke können auch ein Teil des dentalen Objekts sein. Die MRT-Vorrichtung (Magnetresonanztomografie-Vorrichtung) ist eine herkömmliche MRT-Vorrichtung zur Vermessung eines Kopfes, insbesondere eines Oberkiefers und/oder eines Unterkiefers. Die MRT-Vorrichtung weist dabei ein Messvolumen auf, wobei das Objektvolumen des Objekts innerhalb des Messvolumens angeordnet wird, um das Objekt zu vermessen. Nach dem vorliegenden Verfahren werden mehrere MRT-Segmentaufnahmen, also mindestens zwei MRT-Segmentaufnahmen, mittels der MRT-Vorrichtung aufgenommen, wobei jede der MRT-Segmentaufnahmen einen festgelegten Segmentvolumenbereich abbildet. Die Segment-Volumenbereiche können beliebig geformt sein und beispielsweise eine quaderförmige Geometrie aufweisen.

Die Segment-Volumenbereiche der einzelnen MRT-Segmentaufnahmen können auch eine gekrümmte Form aufweisen, wobei in diesem Fall eine besondere MRT-Vorrichtung verwendet wird, die einen solchen gekrümmten Segment-Volumenbereich aufnehmen kann. Bei einer solchen MRT-Vorrichtung können beispielsweise die Gradientenspulen so angeordnet sein, dass die Isolinien eines Gradientenfeldes gekrümmt verlaufen.

Eine MRT-Segmentaufnahme kann eine einzelne MRT-Schichtaufnahme enthalten, so dass die MRT-Segmentaufnahme in einem Schritt mittels der MRT-Vorrichtung aufgenommen wird. Auf diese Weise werden die einzelnen MRT-Segmentaufnahmen nacheinander vermessen. Alternativ dazu kann eine weitere MRT-Vorrichtung verwendet werden, die die MRT-Segmentaufnahmen gleichzeitig aufnehmen kann, wobei eine solche MRT-Vorrichtung die Anregung mehrerer Schichten im Objektvolumen ermöglicht und auf ein sogenanntes Multi-Puls-Anregungs-Verfahren beruht, wie im folgenden Fachartikel beschrieben (Benedikt A. Poser. Simultaneous multislice excitation by parallel transmission. Magn Reson Med. 2014 April; 71 (4): 1416-1427).

Bei einer herkömmlichen MRT-Vorrichtung erfolgt die Vermessung innerhalb des Objektvolumens entlang eines X-Gradienten, entlang eines Y-Gradienten und entlang eines Z-Gradienten, wobei die Auflösung durch die Einstellung einer Länge, einer Breite und einer Tiefe eines Voxels innerhalb des Objektvolumens eingestellt wird, indem die Gradientenspulen entsprechend angesteuert werden. Eine typische Auflösung wird durch ein Voxel mit einer Länge von 0,5 mm, einer Breite von 0,5 mm und einer Tiefe zwischen 1,5 mm und 15 mm bestimmt.

Die Länge und die Breite eines Voxel können zwischen 0,3 mm und 2 mm betragen, so dass durch die Abmessungen des einzelnen Voxels die Auflösung der MRT-Segmentaufnahme bestimmt ist.

Das vorliegende Verfahren kann beispielsweise eine besondere Ansteuerung der MRT-Vorrichtung verwenden, die einen besonderen RF-Puls (2D-Puls) erzeugt, um gekrümmte Volumenbereiche anzuregen, die anschließend als planare Projektionen ausgelesen werden. Hierfür reichen also die technischen Möglichkeiten einer herkömmlichen MRT-Vorrichtung aus.

Das vorliegende Verfahren kann auch als Anregungspuls einen herkömmlichen 1D-RF-Puls verwenden, der gegenüber der 2D-Pulstechnik den Vorteil einer schnellere Bildgebung sowie geringerer Empfindlichkeit gegenüber Magnetfeldstörungen beispielsweise durch Metalle oder Luft aufweist. Solche Störungen durch Metallteile und Luft können insbesondere im Bereich des Oberkiefers und des Unterkiefers auftreten.

Die verwendete MRT-Vorrichtung kann auch auf einem MRT-Verfahren beruhen, bei dem mittels spezieller, zusätzlicher nicht-linearer Gradientenspulen im Meßvolumen räumliche gekrümmte Bereiche gleicher Magnetfeldstärke erzeugt werden, um sowohl gekrümmte Volumenbereiche anzuregen als auch bildgebend auszulesen, wie in EP2511725A1 offenbart.

Das Verfahren kann auch eine herkömmliche MRT-Vorrichtung verwenden, die herkömmliche Gradientenspulen mit geraden Isolinien der Gradientenfelder aufweisen. Dadurch müssen also keine zusätzlichen Gradientenspulen eingebaut werden, so dass sowohl Messvolumen der MRT-Vorrichtung nicht verringert wird als ich höhere Kosten eines Umbaus der MRT-Vorrichtung vermieden werden. Das vorliegende Verfahren kann also mittels einer herkömmlichen MRT-Vorrichtung ohne besondere Anpassungen der MRT-Vorrichtung durchgeführt werden.

Die Segment-Volumenbereiche sind dabei so festgelegt, dass sie sich höchstens teilweise überlappen. Die Segment-Volumenbereiche können also so angeordnet sein, dass sie nur teilweise das Objekt beinhalten und Lücken zwischen den Objekt-Volumenbereichen entstehen oder die Segment-Volumenbereiche können sich teilweise überlappen, so dass zumindest ein Teil des Objekts durchgehend erfasst wird. Die Segment-Volumenbereiche können automatisch festgelegt werden oder durch einen Benutzer festgelegt werden.

Die Segment-Volumenbereiche können sich überlappen jedoch beinhalten sich nicht vollständig gegenseitig. Alternativ können die Segment-Volumenbereiche so angeordnet sein, dass Lücken bei der Vermessung des Objekts entstehen. Die Segment-Volumenbereiche können auch nur einen Teil eines Kiefers abdecken.

Die Segment-Volumenbereiche können auch mit einer unterschiedlichen Auflösung aufgenommen werden, so dass die erzeugten MRT-Segmentaufnahmen ebenfalls unterschiedliche Auflösungen aufweisen.

Die Zielfläche kann zur Durchführung des vorliegenden Verfahrens relativ zur MRT-Vorrichtung bereits festgelegt sein oder computergestützt beziehungsweise durch einen Benutzer festgelegt werden. Aus den einzelnen MRT-Segmentaufnahmen wird das zweidimensionale Kompositbild erzeugt, das dem zweidimensionalen Aggregationsbild durch die Zielfläche entspricht. Das Aggregationsbild entspricht also einer Abbildung der MRT-Segmentaufnahmen auf die Zielfläche.

Die Zielfläche kann beispielsweise dem Verlauf des Oberkiefers und/oder des Unterkiefers folgend angeordnet sein, so dass das Kompositbild einer herkömmlichen Panoramaschichtaufnahme aus der Röntgendiagnostik entspricht oder nachgebildet ist.

In Analogie zu einer Panoramaschichtaufnahme aus der Röntgendiagnostik wird also die Lage und Ausrichtung der scharfen Schicht durch das Festlegen der Zielfläche festgelegt.

Die Zielfläche kann beispielsweise eine Mittelfläche des Objektvolumens sein, die durch die Mittelebenen der einzelnen Segment-Volumenbereiche definiert sein kann oder durch die Mittelfläche einer anatomischen Struktur, wie eines Kiefers, definiert sein kann.

Jede der MRT-Segmentaufnahmen kann eine dreidimensionale Aufnahme oder eine zweidimensionale Aufnahme sein. Falls die MRT-Segmentaufnahme eine dreidimensionale Aufnahme ist, werden die einzelnen Voxel der MRT-Segmentaufnahme in eine Projektionsrichtung, die beispielsweise orthogonal zur Zielfläche angeordnet sein kann, auf die Zielfläche projiziert, um das Kompositbild zu erzeugen. Das einzelne Voxel kann bei der Produktion auch mit einem festgelegten Gewichtungsfaktor gewichtet werden. Falls die einzelne MRT-Segmentaufnahme eine zweidimensionale Aufnahme ist, wird jeder Pixel der MRT-Segmentaufnahme direkt auf die Zielfläche projiziert.

Die zweidimensionale Aufnahme hat also entlang einer der Achsen des jeweiligen Segment-Volumenbereichs nur eine Schicht Voxel, während die dreidimensionale Aufnahme entlang aller Achsen des jeweiligen Segment-Volumenbereichs mehrere Voxel aufweist. Die Projektion erfolgt indem die vorhandenen Voxel (eine Schicht oder mehrerer Schichten) des jeweiligen Segment-Volumenbereichs entlang einer Projektionsrichtung, die beispielsweise senkrecht zur Zielfläche angeordnet sein kann, auf die Zielfläche projiziert und aggregiert werden.

Die Zielfläche kann vor dem Festlegen der Segment-Volumenbereiche im Objektvolumen festgelegt werden oder bereits festgelegt sein, so dass die Segment-Volumenbereiche in Abhängigkeit von der bekannten Zielfläche angeordnet werden. Die Segment-Volumenbereiche können beispielsweise so angeordnet sein, dass die Zielfläche zumindest teilweise darin enthalten ist.

Die Zielfläche kann nach dem Festlegen oder auch nach der Vermessung der Segment-Volumenbereiche nachträglich relativ zur MRT-Vorrichtung innerhalb des Messvolumens und/oder relativ zu den Segment-Volumenbereichen verschoben werden. Die Zielfläche kann beispielsweise in Abhängigkeit von den festgelegten Segment-Volumenbereichen nachträglich angepasst werden.

Ein Vorteil des vorliegenden Verfahrens besteht darin, dass zur Erfassung des dentalen Objekts nur einzelne MRT-Segmentaufnahmen von Segment-Volumenbereichen entlang des Kieferbogens aufgenommen werden müssen, wobei beispielsweise durch die Projektion der MRT-Segmentaufnahmen auf die Zielkurve das Kompositbild zusammengefügt wird. Im Vergleich zum Curved-MPR-Verfahren muss also nicht der gesamte Volumendatensatz der Zahnsituation aufgenommen werden und anschließend ein Volumenbereich für die Panoramakurve manuell aus dem Volumendatensatz ausgewählt werden. Dadurch wird die Aufnahmedauer zur Durchführung des Verfahrens verkürzt.

Vorteilhafterweise kann eine Tiefe jedes der Segment-Volumenbereiche festgelegt sein, wobei jeder Segment-Volumenbereich bezüglich seiner Lage und Ausrichtung innerhalb des Messvolumens festgelegt ist.

Dadurch sind die Lage und die Ausrichtung der einzelnen Segment-Volumenbereiche innerhalb des Messvolumens der MRT-Vorrichtung festgelegt. Die Segment-Volumenbereiche können beispielsweise so angeordnet sein, dass in einem Kinder-Programm automatisch die einzelnen Segment-Volumenbereiche entlang eines Oberkiefers und/oder eines Unterkiefers eines durchschnittlichen Patientenkopfes eines Kindes vermessen werden. In einem Erwachsenen-Programm sind die Segment-Volumenbereiche dann entsprechend so angeordnet, dass die Segment-Volumenbereiche nacheinander entlang eines Oberkiefers und/oder eines Unterkiefers eines durchschnittlichen Patientenkopfes eines Erwachsenen vermessen werden.

Der Benutzer kann dann an der MRT-Vorrichtung ein entsprechendes Programm auswählen.

Vorteilhafterweise kann die Tiefe des Segment-Volumenbereichs zwischen 0,5 mm und 30 mm, vorzugsweise zwischen 1 mm und 15 mm, betragen.

Dadurch ist der Oberkiefer und/oder der Unterkiefer innerhalb des Segment-Volumenbereiches vollständig enthalten und wird demnach vollständig als Projektion im Kompositbild abgebildet.

Die Tiefe eines quaderförmigen Segment-Volumenbereichs kann beispielsweise in einer orthogonalen Richtung relativ zur Zielfläche angeordnet sein. Die Länge und die Breite des Segment-Volumenbereichs kann so gewählt werden, dass das Objekt darin enthalten ist.

Vorteilhafterweise kann jede der MRT-Segmentaufnahmen einen quaderförmigen Segment-Volumenbereich oder einen gekrümmten länglichen Segment-Volumenbereich abbilden.

Die Geometrie des Segment-Volumenbereichs ist durch den Aufbau der MRT-Vorrichtung und insbesondere der Gradientenspulen der MRT-Vorrichtung beeinflusst.

Bei einer herkömmlichen MRT-Vorrichtung werden drei Gradientenspulen verwendet, die lineare Gradientenfelder entlang der x, y, z Achsen einer Patientenöffnung erzeugen. Damit kann durch Überlagerung der Gradientenfelder eine beliebig im Raum drehbare planare Fläche eine eindeutige Resonanzfrequenz erhalten. Der Anregungsbereich mittels herkömmlicher 1D Pulse ist die angeregte planare Fläche extrudiert entlang der drei Flächenachsen und stellt damit einen beliebig im Raum orientierten Quader dar.

Vorteilhafterweise kann die Zielfläche des zweidimensionalen Kompositbildes eine gekrümmte Zielfläche sein, die durch den Oberkiefer und/oder den Unterkiefer eines Musterkopfes oder einer Voraufnahme des Patientenkopfes verläuft. Dadurch wird das zweidimensionale Kompositbild als Schnittbild durch die Zielfläche erstellt, so dass das Kompositbild einer herkömmlichen Panoramaschichtaufnahme aus der Röntgendiagnostik entspricht.

Die Lage und Ausrichtung der gekrümmten Zielfläche innerhalb des Messvolumens der MRT-Vorrichtung wird dabei durch die Anordnung des Oberkiefers und/oder des Unterkiefers des Musterkopfes (eines Kindes oder eines Erwachsenen) oder durch die Voraufnahme des Patientenkopfes vorgegeben. Die MRT-Vorrichtung wird also schrittweise so eingestellt, dass die festgelegten Segment-Volumenbereiche nacheinander vermessen werden und die MRT-Segmentaufnahmen auf die festgelegte Zielfläche projiziert werden.

Die Voraufnahme des Patientenkopfes kann beispielsweise eine dreidimensionale Röntgenaufnahme oder eine dreidimensionale MRT-Aufnahme des Patientenkopfes des jeweiligen Patienten sein.

Die Voraufnahme des Patientenkopfes kann auch eine zweidimensionale Röntgenaufnahme oder eine zweidimensionale MRT-Aufnahme sein, die die Positionierung der Zielfläche ermöglicht.

Die Zielfläche wird also unter Verwendung eines Musterkopfes (3D-Modell eines Kopfes) oder einer Voraufnahme des Patientenkopfes (patientenbezogene Planungsaufnahme) festgelegt. Die Fixierung des Patienten relativ zur MRT-Vorrichtung kann beispielsweise mittels einer Kopfhalterung und/oder eine Aufbisshalterung erfolgen.

Vorteilhafterweise kann die Zielfläche in einer Richtung senkrecht zu einer Okklusionsebene des Oberkiefers und/oder des Unterkiefers eben geformt sein oder in einer Richtung gekrümmt dem Verlauf von Zahnachsen des Oberkiefers und/oder des Unterkiefers folgend geformt sein.

Dadurch ist die Zielfläche bei der ersten Alternative entlang des Oberkiefers gekrümmt geformt und in der Richtung senkrecht zu der Okklusionsebene eben geformt. Bei der zweiten Alternative ist die Zielfläche sowohl in einer approximalen Richtung entlang des Verlaufs des Kiefers als auch in der okklusalen Richtung der Zahnachsen folgend gekrümmt geformt. Dadurch wird bei der zweiten Alternative ein Kompositbild durch die Zielfläche erzeugt, das den Oberkiefer und/oder den Unterkiefer besonders deutlich abbildet.

Die Okklusionsebene beschreibt die räumliche Ebene, auf der sich die Zähne des Ober- und Unterkiefers treffen. Konstruiert wird sie durch die Verbindungslinien zwischen Inzisalpunkt (Berührungspunkt der Schneidekanten der Zähne 31 und 41) und dem distobukkalen Höcker der Zähne 36 und 41 und verläuft in der Regel durch die Lippenschlusslinie.

Als Zahnachse wird die Zahnlängsachse eines Zahnes bezeichnet. Sie ist als Verbindungslinie zwischen der Wurzelspitze bei einwurzeligen Zähnen und der Mitte der Schneidekante und bei mehrwurzligen Zähnen zwischen der Wurzelgabelung (Bifurkation, Trifurkation) und der der Kauflächenmitte definiert. Die Zahnachsen der Frontzähne (Eckzähne und Schneidezähne) im Oberkiefer sind in Normalstellung nach distal geneigt.

Vorteilhafterweise kann das Kompositbild einer herkömmlichen Röntgen-Panoramaschichtaufnahme entsprechen.

Vorteilhafterweise kann die Zielfläche des Kompositbildes dem Verlauf einer scharfen Schicht einer herkömmlichen Röntgen-Panoramaschichtaufnahme entsprechen oder nachgebildet sein.

Dadurch entspricht das Kompositbild einer herkömmlichen Röntgen-Panoramaschichtaufnahme und erleichtert dem Zahnarzt eine Diagnose, wie gewohnt anhand einer Panoramaschichtaufnahme, durchzuführen.

Vorteilhafterweise kann das Kompositbild aus den einzelnen MRT-Segmentaufnahmen erzeugt werden, indem für jedes Pixel des Kompositbildes auf der Zielfläche die Lage des Mittelpunktes dieses Pixels bestimmt wird, wobei für jedes Voxel bzw. Pixel einer der MRT-Segmentaufnahmen der Mittelpunkt bestimmt wird, wobei dieser Mittelpunkt entlang einer bekannten Projektionsrichtung der jeweiligen MRT-Segmentaufnahme auf die Zielfläche projiziert wird, um einen projizierten Mittelpunkt zu ermitteln, wobei für einen Kompositbild-Pixel des Kompositbildes derjenige Voxel bzw. Pixel einer der MRT-Segmentaufnahmen verwendet wird, dessen projizierten Mittelpunkt am nächsten zum Mittelpunkt des jeweiligen Kompositbild-Pixels angeordnet ist.

Die Zielfläche kann also durch die Mittelebenen der MRT-Segmentaufnahmen verlaufen und an den Schnittlinien der Mittelebene einer MRT-Segmentaufnahme mit der jeweils benachbarten MRT-Segmentaufnahme auf die benachbarte Mittelebene wechselt. Wird auf Basis dieser Zielflächendefinition jedem Kompositbild-Pixel genau ein bestimmtes Voxel einer der MRT-Segmentaufnahme zugeordnet, können dadurch an den Übergängen zwischen den Mittelebenen der einzelnen MRT-Segmentaufnahmen im Kompositbild aufgrund der unterschiedlichen Ausrichtungen der MRT-Segmentaufnahmen visuell abrupte Veränderungen der Pixelwerte ("Knicke") entstehen. Ein Vorteil dieser Ausführungsform besteht darin, dass eine einzelne MRT-Segmentaufnahme nicht verzerrt wird und die anatomischen Strukturen innerhalb der MRT-Segmentaufnahme maßstabsgetreu im Kompositbild wiedergegeben werden.

Vorteilhafterweise kann derjenige Pixel bzw. Voxel einer MRT-Segmentaufnahme als der Kompositbild-Pixel verwendet werden dessen Abstand zwischen dem projizierten Mittelpunkt dieses Pixel bzw. Voxel und dem Mittelpunkt des Kompositbild-Pixels kleiner als ein festgelegter Toleranzwert ist und/oder falls ein Winkel zwischen einer gewünschten Projektionsrichtung des Kompositbild-Pixels und einer Projektionsrichtung dieses Pixels bzw. Voxels kleiner als ein festgelegter Maximalwinkel ist.

Die gewünschte Projektionsrichtung des Kompositbild-Pixels kann beispielsweise orthogonal zur Zielfläche angeordnet sein. Die tatsächliche Projektionsrichtung des Pixels bzw. des Voxels ist dabei durch die Orientierung der MRT-Segmentaufnahme, insbesondere durch die Richtung des Z-Gradienten, vorgegeben.

Alternativ können bei der Erzeugung des Kompositbildes aus den einzelnen MRT-Segmentaufnahmen die MRT-Segmentaufnahmen auf die Zielfläche projiziert werden, indem für jedes Pixel des Kompositbildes auf der Zielfläche die Lage eines Mittelpunktes dieses Pixels bestimmt wird, wobei für jedes Pixel bzw. Voxel einer der MRT-Segmentaufnahmen ein Mittelpunkt bestimmt wird, wobei dieser Mittelpunkt entlang einer bekannten Projektionsrichtung der jeweiligen MRT-Segmentaufnahme auf die Zielfläche projiziert wird, um einen projizierten Mittelpunkt zu ermitteln, wobei ein Kompositbild-Pixel des Kompositbildes durch Interpolation von mindestens zwei benachbarten projizierten Pixeln bzw. Voxeln der MRT-Segmentaufnahmen erfolgt.

Bei dieser alternativen Ausführungsform wird das Kompositbild durch Interpolation von benachbarten Pixeln der MRT-Segmentaufnahmen erzeugt. Ein Vorteil dieser alternativen Ausführungsformen besteht darin, dass an den Übergängen zwischen den MRT-Segmentaufnahmen visuell weniger auffällige Übergänge, also kleinere Knicke, entstehen.

Vorteilhafterweise kann vor der Erstellung bzw. Vermessung der MRT-Segmentaufnahmen die Anzahl der MRT-Segmentaufnahmen, die Lage und/oder die Ausrichtung der MRT-Segmentaufnahmen relativ zur MRT-Vorrichtung mittels eines Computers automatisch oder teilweise automatisch festgelegt werden.

Dadurch werden die Anzahl, die Lage und die Ausrichtung der MRT-Segmentaufnahmen automatisch mittels des Computers bestimmt. Falls das Objekt beispielsweise die linke Seite des Oberkiefers ist, können zwei oder drei Segment-Volumenbereiche automatisch bestimmt werden, die sich teilweise überlappen und den linken Oberkiefer vollständig enthalten.

Bei der automatischen Planung kann das Objektvolumen, beinhaltend den Oberkiefer und/oder den Unterkiefer automatisch in 3-30 Segment-Volumenbereiche, vorzugsweise 6-8 Segment-Volumenbereiche, aufgeteilt werden. Die Segment-Volumenbereiche werden dabei so zueinander angeordnet, dass sie entlang des Oberkiefers bzw. des Unterkiefers gleichmäßig verteilt sind und eine vollständige Erfassung des Objektvolumens ermöglichen.

Vorteilhafterweise kann jeder der Segment-Volumenbereiche innerhalb des Objektvolumens so angeordnet werden, dass eine Mittelebene des jeweiligen Segment-Volumenbereichs parallel zu einer Tangente der Zielfläche angeordnet ist.

Dadurch werden die einzelnen Segment-Volumenbereiche in Abhängigkeit von der bereits festgelegten Zielfläche so angeordnet, dass die jeweilige Mittelebene des Segment-Volumenbereichs tangential zur Zielfläche angeordnet ist. Die Mittelebene eines Segment-Volumenbereichs kann beispielsweise einer Vorzugsrichtung der jeweiligen MRT-Segmentaufnahme entsprechen, die durch eine längste Kante bzw. eine größte Seitenfläche des quaderförmigen Segment-Volumenbereichs vorgegeben sein kann.

Vorteilhafterweise kann jeder der Segment-Volumenbereiche so angeordnet werden, dass eine Richtung der geringsten Auflösung der jeweiligen MRT-Segmentaufnahme orthogonal zur Zielfläche angeordnet ist.

Dadurch werden die MRT-Segmentaufnahmen in Richtung der geringsten Auflösung auf die Zielfläche projiziert, so dass das zweidimensionale Kompositbild eine höhere Auflösung aufweist.

Bei einer herkömmlichen MRT-Vorrichtung erfolgt die Vermessung innerhalb des Objektvolumens entlang eines X-Gradienten, entlang eines Y-Gradienten und entlang eines Z-Gradienten, wobei die Auflösung durch die Einstellung einer Länge, einer Breite und einer Tiefe eines Voxels innerhalb des Objektvolumens eingestellt wird. Eine typische Auflösung wird durch ein Voxel mit einer Länge von 0,5 mm, einer Breite von 0,5 mm und einer Tiefe zwischen 1,5 mm und 15 mm bestimmt. Die Richtung der Tiefe des Voxels stimmt demnach mit der Richtung der geringsten Auflösung der jeweiligen MRT-Segmentaufnahme überein.

Vorteilhafterweise kann mittels eines Optimierungsverfahrens die Anzahl, die Lage und/oder die Ausrichtung der Segment-Volumenbereiche solange variiert werden, bis ein Fehlermaß einen festgelegten Toleranzwert unterschreitet.

Dadurch werden die Anzahl, die Lage und die Ausrichtung der Segment-Volumenbereiche innerhalb des Messvolumens der MRT-Vorrichtung mittels des Optimierungsverfahrens automatisch bestimmt. Dadurch wird die Dauer der Planung der einzelnen Segment-Volumenbereiche verkürzt.

Vorteilhafterweise kann als Fehlermaß eine Summe der Winkel zwischen einer tatsächlichen Projektionsrichtung und einer gewünschten Projektionsrichtung der einzelnen Kompositbild-Pixel des Kompositbildes verwendet werden, wobei die gewünschte Projektionsrichtung einer Normalen relativ zur Zielfläche entspricht, wobei die tatsächliche Projektionsrichtung durch die Projektionsrichtung des verwendeten Pixels bzw. Voxels aus der jeweiligen MRT-Segmentaufnahme festgelegt ist.

Dadurch wird durch das Optimierungsverfahren gewährleistet, dass der festgelegte Toleranzwert des Fehlermaßes nicht überschritten wird. Denn falls die Anzahl der Segment-Volumenbereiche zu gering ist, können an den Übergangsbereichen zwischen den einzelnen MRT-Segmentaufnahmen in dem Kompositbild deutliche Knicke oder Verzerrungen entstehen, da insbesondere an den Übergangsbereichen die tatsächliche Projektionsrichtung von der gewünschten Projektionsrichtung deutlich abweicht.

Vorteilhafterweise kann vor der Erstellung der MRT-Segmentaufnahmen die Anzahl der MRT-Segmentaufnahmen, die Lage und/oder die Ausrichtung der MRT-Segmentaufnahmen relativ zur MRT-Vorrichtung durch einen Benutzer festgelegt werden, wobei mittels eines virtuellen Werkzeugs die Lage und die Ausrichtung der Segment-Volumenbereiche der MRT-Segmentaufnahmen relativ zur MRT-Vorrichtung festgelegt werden.

Dadurch können die einzelnen Segment-Volumenbereiche durch den Benutzer virtuell festgelegt werden.

Die Festlegung der Segment-Volumenbereiche kann auch teilweise automatisch erfolgen, wobei im ersten Schritt eine vorläufige Lage und Ausrichtung der Segment-Volumenbereiche vorgeschlagen wird und im zweiten Schritt durch den Benutzer mittels eines virtuellen Werkzeugs korrigiert werden kann.

Das virtuelle Werkzeug kann dabei ein beliebiger Computeralgorithmus sein, der eine Interaktion zwischen einem Benutzer und der Segment-Volumenbereiche ermöglicht.

Vorteilhafterweise kann ein Musterkopf mit einem Muster-Oberkiefer und/oder einem Muster-Unterkiefer schematisch mittels einer Anzeigevorrichtung dargestellt werden, wobei die festgelegten Segment-Volumenbereiche und deren Anordnung relativ zum Musterkopf und/oder die festgelegte Zielfläche relativ zum Musterkopf graphisch dargestellt werden.

Dadurch wird dem Benutzer graphisch deutlich gemacht, an welchen Stellen die Segment-Volumenbereiche und die festgelegte Zielfläche relativ zum Musterkopf liegen. Dadurch wird es dem Benutzer erleichtert, das erzeugte Kompositbild zu beurteilen.

Die Übergänge zwischen den MRT-Segmentaufnahmen können ebenfalls mittels der Anzeigevorrichtung visuell dargestellt werden.

Das erzeugte Kompositbild kann auch in ein zweidimensionales oder dreidimensionales Modell eines Musterkopfes oder eine patientenbezogenen Voraufnahme eingeblendet werden, um dem Benutzer die Orientierung relativ zu den Strukturen außerhalb des Kiefers, wie dem Schädel, zu ermöglichen. Der Bereich an dem das Kompositbild eingeblendet wird, kann aus dem Modell des Musterkopfes bzw. der patientenbezogene Voraufnahme virtuell ausgestanzt werden, so dass das Kompositbild nicht mit dem Hintergrund überlagert wird.

Vorteilhafterweise können mittels einer Anzeigevorrichtung das erzeugte Kompositbild und der Musterkopf mit der grafischen Darstellung der einzelnen Segment-Volumenbereiche und/oder mit der festgelegten Zielfläche (22) gleichzeitig dargestellt werden.

Dadurch kann der Benutzer sich anhand des Musterkopfes leichter orientieren und dadurch das erzeugte Kompositbild besser diagnostizieren.

Vorteilhafterweise kann jede MRT-Segmentaufnahme aus einer einzelnen MRT-Schichtaufnahme bestehen oder aus einem Stapel mehrerer MRT-Schichtaufnahmen bestehen.

Dadurch kann also die MRT-Segmentaufnahme aus einem einzelnen Stapel mehrerer MRT-Schichtaufnahmen gebildet sein, so dass die MRT-Segmentaufnahme entweder in einem Schritt mittels der MRT-Vorrichtung oder in mehreren nacheinander folgenden Schritten aufgenommen wird.

Vorteilhafterweise können die MRT-Schichtaufnahmen eines Stapels parallel zueinander senkrecht zu einer festgelegten Aufnahmerichtung angeordnet sein.

Dadurch wird jede der MRT-Schichtaufnahmen Schicht für Schicht nacheinander vermessen.

Vorteilhafterweise kann bei allen MRT-Segmentaufnahmen die räumliche Lage des dentalen Objekts relativ zum Meßvolumen der MRT-Vorrichtung gleich bleiben.

Das dentale Objekt, wie ein Patientenkopf, wird also relativ zur MRT-Vorrichtung fest positioniert, wobei anschließend die einzelnen MRT-Segmentaufnahmen aufgenommen werden. Bei einem bekannten Verfahren, beispielsweise zur Vermessung der Wirbelsäule, werden mehrerer MRT-Aufnahmen durchgeführt, wobei der Patient zwischen den MRT-Aufnahmen relativ zur MRT-Vorrichtung verschoben wird.

Ein Vorteil einer festen Positionierung des Objekts relativ zur MRT-Vorrichtung besteht darin, dass die Lage des Objekts relativ zur MRT-Vorrichtung in allen MRT-Segmentaufnahmen exakt gleich bleibt und bei der Verrechnung der verschiedenen Aufnahmen keine durch die Verschiebung des Patienten verursachten zusätzlichen räumlichen Ungenauigkeiten entstehen. Weiterhin wird eine Verlängerung der Gesamtaufnahmedauer durch die mechanische Verlagerung vermieden sowie der Workflow für den MRT-Arbeiter einfach gehalten.

### Kurzbeschreibung der Zeichnungen

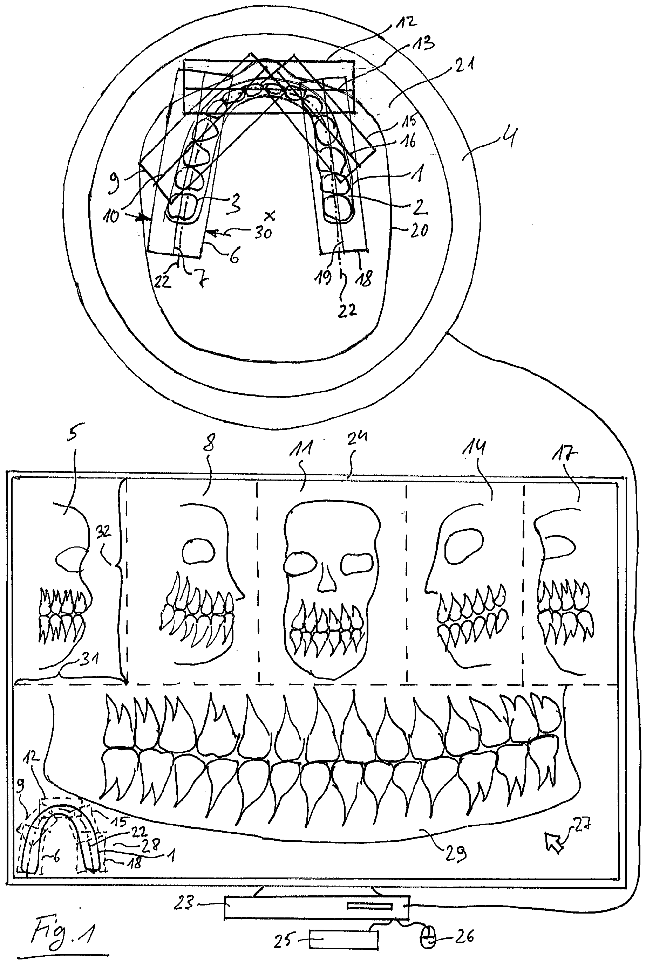

Die Erfindung wird anhand der Zeichnungen erläutert. Es zeigt, die
- Fig. 1: eine Skizze zur Verdeutlichung des vorliegen-den Verfahrens.

### Ausführungsbeispiele

Die Fig. 1 zeigt eine Skizze zur Verdeutlichung des vorliegenden Verfahrens zur Erfassung eines dentalen Objekts 1, insbesondere zumindest eines Teils eines Oberkiefers 2 und/oder eines Unterkiefers 3 mittels einer MRT-Vorrichtung 4. Dabei wird eine erste MRT-Segmentaufnahme 5 eines ersten Segment-Volumenbereichs 6 mit einer Mittelebene 7, eine zweite MRT-Segmentaufnahme 8 eines zweiten Segment-Volumenbereichs 9 mit einer Mittelebene 10, eine dritte MRT-Segmentaufnahme 11 eines dritten Segment-Volumenbereichs 12 mit einer Mittelebene 13, eine vierte MRT-Segmentaufnahme 14 eines vierten Segment-Volumenbereichs 15 mit einer Mittelebene 16 und eine fünfte MRT-Segmentaufnahme 17 eines fünften Segment-Volumenbereichs 18 mit einer Mittelebene 19 aufgenommen. Die MRT-Segmentaufnahmen 5,8, 11,14 und 17 bilden dabei die Segment-Volumenbereiche 6, 9, 12, 18 ab, die sich teilweise überlappen. Zur Erfassung des dentalen Objekts 1 wird ein Patientenkopf 20 relativ zur MRT-Vorrichtung 4 innerhalb eines Messvolumens 21 der MRT-Vorrichtung 4 beispielsweise mittels einer Kopfhalterung und/oder mittels einer Aufbisshalterung positioniert. Anschließend werden die einzelnen MRT-Segmentaufnahmen 5,8,11, 14 und 17 nacheinander mittels der MRT-Vorrichtung 4 durchgeführt. Eine gekrümmte Zielfläche 22 relativ zur MRT-Vorrichtung innerhalb des Messvolumens 21 ist bereits festgelegt oder wird durch den Benutzer festgelegt. Die Zielfläche 22 ist als eine Strich-Punkt-Linie dargestellt und verläuft als eine Mittelebene durch den Oberkiefer 2 und/oder den Unterkiefer 3. Die Zielfläche 22 kann auch in einer okklusalen Richtung gekrümmt sein, so dass die Zielfläche 22 einer gemittelten Fläche durch die Zahnachsen der einzelnen Zähne der Kiefer 2 und 3 entspricht. Die Bilddaten der MRT-Segmentaufnahmen 5, 8, 11, 14 und 17 werden von der MRT-Vorrichtung 4 an einen Computer 23 übermittelt und werden mittels einer Anzeigevorrichtung 24, wie eines Monitors, graphisch dargestellt. An den Computer 23 sind Eingabemittel, wie eine Tastatur 25 und eine Maus 26, angeschlossen. Die Eingabemittel 25 und 26 erlauben dem Benutzer ein virtuelles Werkzeug mittels eines Cursors 27 zu bedienen. Mittels der Anzeigevorrichtung 24 werden in einer schematischen Darstellung 28 die festgelegten Segment-Volumenbereiche 6, 9, 12, 15 und 18 sowie die Zielfläche 22 relativ zum Objekt 1 schematisch dargestellt. Die schematische Darstellung 28 erlaubt es dem Benutzer, wie einem Zahnarzt, die Lage der Segment-Volumenbereiche und der Zielfläche 22 relativ zum Objekt besser zu beobachten. Aus den einzelnen MRT-Segmentaufnahmen 5, 8, 11, 14 und 17 wird mittels des Computers 23 ein zweidimensionales Kompositbild 29 erzeugt, wobei die MRT-Segmentaufnahmen 5, 8, 11, 14 und 17 auf die Zielfläche 22 projiziert werden. Das Kompositbild 29 entspricht dabei einer herkömmlichen Panoramaschichtaufnahme aus der Röntgendiagnostik, wobei die Zielfläche 22 den Verlauf der scharfen Schicht einer herkömmlichen Panoramaschichtaufnahme entspricht. Die Tiefe 30 des ersten Segmentvolumenbereichs 6 beträgt im vorliegenden Fall 25 mm. Eine Breite 31 des ersten Segment-Volumenbereichs 6, die der Breite der ersten MRT-Segmentaufnahme 5 entspricht, beträgt 100 mm. Eine Länge 32 des ersten Segment-Volumenbereichs 6, die der Länge der ersten MRT-Segmentaufnahme 5 entspricht, beträgt 250 mm.

### Bezugszeichen

- 1: Halterung
- 2: Oberkiefer
- 3: Unterkiefer
- 4: MRT-Vorrichtung
- 5: erste MRT-Segmentaufnahme
- 6: Segment-Volumenbereich
- 7: Mittelebene
- 8: zweite MRT-Segmentaufnahme
- 9: zweiter Segmentvolumenbereich
- 10: Mittelebene
- 11: dritte MRT-Segmentaufnahme
- 12: dritter Segmentvolumenbereich
- 13: Mittelebene
- 14: vierte MRT-Segmentaufnahme
- 15: vierter Segmentvolumenbereich
- 16: Mittelebene
- 17: fünfte MRT-Segmentaufnahme
- 18: fünfter Segmentvolumenbereich
- 19: Mittelebene
- 20: Patientenkopf
- 21: Messvolumen
- 22: gekrümmte Zielfläche
- 23: Computer
- 24: Monitor bzw. Anzeigevorrichtung
- 25: Tastatur
- 26: Maus
- 27: Cursor
- 28: eine schematische Darstellung
- 29: Kompositbild
- 30: Tiefe
- 31: Breite
- 32: Länge

## Patentansprüche

1. Verfahren zur Erfassung eines dentalen Objekts (1) mit einem Objektvolumen, insbesondere zumindest eines Teils eines Oberkiefers (2) und/oder eines Unterkiefers (3), mittels einer MRT-Vorrichtung (4), wobei mehrere MRT-Segmentaufnahmen (5, 8, 11, 14, 17) mittels der MRT-Vorrichtung (4) innerhalb eines Messvolumens (21) der MRT-Vorrichtung (4) aufgenommen werden, die jeweils festgelegte Segment-Volumenbereiche (6, 9, 12, 15, 18) abbilden, wobei die Segment-Volumenbereiche (6, 9, 12, 15, 18) sich höchstens teilweise überlappen, wobei eine Zielfläche (22) festgelegt wird oder festgelegt ist, wobei computergestützt aus den einzelnen MRT-Segmentaufnahmen (5, 8, 11, 14, 17) ein zweidimensionales Kompositbild (29) erzeugt wird, das einem zweidimensionalen Aggregationsbild durch die Zielfläche (22) innerhalb des Objektvolumens des dentalen Objekts (1) entspricht, wobei vor der Erstellung der MRT-Segmentaufnahmen (5, 8, 11, 14, 17) die Anzahl der MRT-Segmentaufnahmen (5, 8, 11, 14, 17), die Lage und/oder die Ausrichtung der MRT-Segmentaufnahmen (5, 8, 11, 14, 17) relativ zur MRT-Vorrichtung (4) mittels eines Computers (23) automatisch oder teilweise automatisch festgelegt werden, wobei jeder der Segment-Volumenbereiche (6, 9, 12, 15, 18) innerhalb des Objektvolumens so angeordnet wird, dass eine Mittelebene (7, 10, 13, 16, 19) des jeweiligen Segment-Volumenbereichs (6, 9, 12, 15, 18) parallel zu einer Tangente der Zielfläche (22) angeordnet ist, wobei mittels eines Optimierungsverfahrens die Anzahl, die Lage und/ oder die Ausrichtung der Segment-Volumenbereiche (6, 9, 12, 15, 18) solange variiert werden, bis ein Fehlermaß einen festgelegten Toleranzwert unterschreitet.

2. Verfahren nach Anspruch 1, **dadurch gekennzeichnet, dass** eine Tiefe (30) jedes der Segment-Volumenbereiche (6, 9, 12, 15, 18) festgelegt ist, wobei jeder Segment-Volumenbereich (6, 9, 12, 15, 18) bezüglich seiner Lage und Ausrichtung innerhalb des Messvolumens (21) festgelegt ist.

3. Verfahren nach Anspruch 2, **dadurch gekennzeichnet, dass** die Tiefe (30) des Segment-Volumenbereichs (6, 9, 12, 15, 18) zwischen 0,5 mm und 30 mm, vorzugsweise zwischen 1 mm und 15 mm, beträgt.

4. Verfahren nach einem der Ansprüche 1 bis 3, **dadurch gekennzeichnet, dass** die Zielfläche (22) des zweidimensionalen Kompositbildes (29) eine gekrümmte Zielfläche ist, die durch den Oberkiefer und/oder den Unterkiefer eines Musterkopfes oder einer Voraufnahme des Pateientenkopfes verläuft.

5. Verfahren nach Anspruch 4, **dadurch gekennzeichnet, dass** die Zielfläche (22) in einer Richtung senkrecht zu einer Okklusionsebene des Oberkiefers (2) und/oder des Unterkiefers (3) eben geformt ist oder in einer Richtung gekrümmt dem Verlauf von Zahnachsen des Oberkiefers (2) und/oder des Unterkiefers (3) folgend geformt ist.

6. Verfahren nach Anspruch 4 oder 5, **dadurch gekennzeichnet, dass** das Kompositbild (29) einer herkömmlichen Röntgen-Panoramaschichtaufnahme entspricht oder nachgebildet ist.

7. Verfahren nach einem der Ansprüche 1 bis 6, **dadurch gekennzeichnet, dass** die Zielfläche (22) des Kompositbildes (29) dem Verlauf einer scharfen Schicht einer herkömmlichen Röntgen-Panoramaschichtaufnahme entspricht oder nachgebildet ist.

8. Verfahren nach einem der Ansprüche 1 bis 7, **dadurch gekennzeichnet, dass** jeder der Segment-Volumenbereiche (6, 9, 12, 15, 18) so angeordnet wird, dass eine Richtung der geringsten Auflösung der jeweiligen MRT-Segmentaufnahme orthogonal zur Zielfläche (22) angeordnet ist.

9. Verfahren nach einem der Ansprüche 1 bis 8, **dadurch gekennzeichnet, dass** vor der Erstellung der MRT-Segmentaufnahmen (5, 8, 11, 14, 17) die Anzahl der MRT-Segmentaufnahmen (5, 8, 11, 14, 17), die Lage und/oder die Ausrichtung der MRT-Segmentaufnahmen (5, 8, 11, 14, 17) relativ zur MRT-Vorrichtung (4) durch einen Benutzer festgelegt werden, wobei mittels eines virtuellen Werkzeugs die Lage und die Ausrichtung der Segment-Volumenbereiche (6, 9, 12, 15, 18) der MRT-Segmentaufnahmen relativ zur MRT-Vorrichtung (4) festgelegt werden.

10. Verfahren nach einem der Ansprüche 1 bis 9, **dadurch gekennzeichnet, dass** ein Musterkopf mit einem Muster-Oberkiefer und/oder einem Muster-Unterkiefer schematisch mittels einer Anzeigevorrichtung (24) dargestellt wird, wobei die festgelegten Segment-Volumenbereiche (6, 9, 12, 15, 18) und deren Anordnung relativ zum Musterkopf und/oder die festgelegte Zielfläche (22) relativ zum Musterkopf anhand einer graphischen Darstellung (28) dargestellt werden.

11. Verfahren nach Anspruch 10, **dadurch gekennzeichnet, dass** mittels der Anzeigevorrichtung (24) das erzeugte Kompositbild (29) und der Musterkopf mit der grafischen Darstellung der einzelnen Segment-Volumenbereiche (5, 8, 11, 14, 17) und/oder mit der festgelegten Zielfläche (22) gleichzeitig dargestellt werden.

12. Verfahren nach einem der Ansprüche 1 bis 11, **dadurch gekennzeichnet, dass** jede MRT-Segmentaufnahme (5, 8, 11, 14, 17) aus einer einzelnen MRT-Schichtaufnahme besteht oder aus einem Stapel mehrerer MRT-Schichtaufnahmen besteht.

13. Verfahren nach einem der Ansprüche 1 bis 12, **dadurch gekennzeichnet, dass** bei allen MRT-Segmentaufnahmen (5, 8, 11, 14, 17) die räumliche Lage des dentalen Objekts (1) relativ zum Meßvolumen (21) der MRT-Vorrichtung (4) gleich bleibt.

## Claims

1. A method for detecting a dental object (1) having an object volume, in particular of at least a part of an upper jaw (2) and/or a lower jaw (3), using an MRI device (4), wherein multiple MRI segment images (5, 8, 11, 14, 17) are recorded by means of the MRI device (4) within a measurement volume (21) of the MRI device (4), which represent respectively defined segment volume areas (6, 9, 12, 15, 18), whereby the segment volume areas (6, 9, 12, 15, 18) at most partially overlap, wherein a target area (22) is or will be defined, wherein a two-dimensional composite image (29) is generated in a computer-aided manner from the individual MRI segment images (5, 8, 11, 14, 17), said composite image corresponding to a two-dimensional aggregation image through the target area (22) within the object volume of the dental object (1), wherein before the MRI segment images (5, 8, 11, 14, 17) are created the number of MRI segment images (5, 8, 11, 14, 17), the position and/or the orientation of the MRI segment images (5, 8, 11, 14, 17) relative to the MRI device (4) are defined automatically or semi-automatically by means of a computer (23), each of the segment volume areas (6, 9, 12, 15, 18) being arranged within the object volume such that a central plane (7, 10, 13, 16, 19) of the respective segment volume area (6, 9, 12, 15, 18) is arranged parallel to one tangent of the target surface (22), wherein the number, the position and/or the orientation of the segment volume areas (6, 9, 12, 15, 18) is varied by means of an optimisation method until an error measure falls below a defined tolerance value.

2. The method according to claim 1, **characterised in that** a depth (30) of each of the segment volume areas (6, 9, 12, 15, 18) is defined, each segment volume area (6, 9, 12, 15, 18) being defined with respect to its position and orientation within the measurement volume (21).

3. The method according to claim 2, **characterised in that** the depth (30) of the segment volume area (6, 9, 12, 15, 18) is between 0.5 mm and 30 mm, preferably between 1 mm and 15 mm.

4. The method according to any one of claims 1 to 3, **characterised in that** the target surface (22) of the two-dimensional composite image (29) is a curved target surface which extends through the upper jaw and/or the lower jaw of a model head or a preview image of the patient's head.

5. The method according to claim 4, **characterised in that** the target surface (22) is planar in form in a direction perpendicular to an occlusal plane of the upper jaw (2) and/or the lower jaw (3) or is curved in form in one direction to follow the course of tooth axes of the upper jaw (2) and/or the lower jaw (3).

6. The method according to claim 4 or 5, **characterised in that** the composite image (29) corresponds to a conventional X-ray panoramic tomographic image or replicates the same.

7. The method according to any one of claims 1 to 6, **characterised in that** the target surface (22) of the composite image (29) corresponds to the course of a sharp layer of a conventional X-ray panoramic tomographic image or replicates the same.

8. The method according to any one of claims 1 to 7, **characterised in that** each of the segment volume areas (6, 9, 12, 15, 18) is arranged such that a direction of the lowest resolution of the respective MRI segment image is arranged orthogonally to the target surface (22).

9. The method according to any one of claims 1 to 8, **characterised in that** before the creation of the MRI segment images (5, 8, 11, 14, 17) the number of MRI segment images (5, 8, 11, 14, 17), the position and/or the orientation of the MRI segment images (5, 8, 11, 14, 17) relative to the MRI device (4) is defined by a user, wherein the position and the orientation of the segment volume areas (6, 9, 12, 15, 18) of the MRI segment images relative to the MRI device (4) are defined by means of a virtual tool.

10. The method according to any one of claims 1 to 9, **characterised in that** a model head with a model upper jaw and/or a model lower jaw is shown schematically by means of a display device (24), wherein the defined segment volume areas (6, 9, 12, 15, 18) and their arrangement relative to the model head and/or the defined target area (22) relative to the model head are shown on the basis of a graphical representation (28).

11. The method according to claim 10, **characterised in that** by means of the display device (24) the generated composite image (29) and the model head with the graphical representation of the individual segment volume areas (5, 8, 11, 14, 17) and/or with the defined target area (22) can be displayed simultaneously.

12. The method according to any one of claims 1 to 11, **characterised in that** each MRI segment image (5, 8, 11, 14, 17) consists of a single MRI tomography or a stack of a plurality of MRI tomographies.

13. The method according to any one of claims 1 to 12, **characterised in that** in all MRI segment images (5, 8, 11, 14, 17) the spatial position of the dental object (1) relative to the measurement volume (21) of the MRI device (4) remains the same.

## Revendications

1. Procédé de détection d'un objet dentaire (1) comportant un volume d'objet, en particulier au moins une partie d'un maxillaire supérieur (2) et/ou d'un maxillaire inférieur (3), au moyen d'un dispositif d'IRM (4), dans lequel plusieurs enregistrements de segments IRM (5, 8, 11, 14, 17) sont enregistrés au moyen du dispositif d'IRM (4) dans un volume de mesure (21) du dispositif d'IRM (4), lesquels représentent respectivement des zones de volume de segment (6, 9, 12, 15, 18) définies, dans lequel les zones de volume de segment (6, 9, 12, 15, 18) se chevauchent au plus partiellement, dans lequel une surface cible (22) est déterminée, dans lequel une image composite (29) bidimensionnelle est générée de manière assistée par ordinateur à partir des enregistrements de segment IRM (5, 8, 11, 14, 17) individuels, ce qui correspond à une image d'agrégation bidimensionnelle à travers la surface cible (22) dans le volume d'objet de l'objet dentaire (1), dans lequel avant la création d'enregistrements de segment IRM (5, 8, 11, 14, 17), le nombre d'enregistrements de segment IRM (5, 8, 11, 14, 17), la position et/ou l'orientation des enregistrements de segment IRM (5, 8, 11, 14, 17) par rapport au dispositif d'IRM (4) sont déterminés automatiquement ou de manière partiellement automatique au moyen d'un ordinateur (23), dans lequel les zones de volume de segment (6, 9, 12, 15, 18) respectives sont disposées dans le volume d'objet de telle sorte qu'un plan central (7, 10, 13, 16, 19) de la zone de volume de segment (6, 9, 12, 15, 18) respective est disposé parallèlement à une tangente de la surface cible (22), dans lequel au moyen d'un procédé d'optimisation, le nombre, la position et/ou l'orientation des zones de volume de segment (6, 9, 12, 15, 18) sont modifiés jusqu'à ce qu'une cote d'erreur soit inférieure à une valeur de tolérance définie.

2. Procédé selon la revendication 1, **caractérisé en ce qu'**une profondeur (30) des zones de volume de segment (6, 9, 12, 15, 18) respectives est définie, la zone de volume de segment (6, 9, 12, 15, 18) respective étant définie par rapport à sa position et son orientation dans le volume de mesure (21).

3. Procédé selon la revendication 2, **caractérisé en ce que** la profondeur (30) de la zone de volume de segment (6, 9, 12, 15, 18) est comprise entre 0,5 mm et 30 mm, de préférence entre 1 mm et 15 mm.

4. Procédé selon l'une quelconque des revendications 1 à 3, **caractérisé en ce que** la surface cible (22) de l'image composite (29) bidimensionnelle est une surface cible incurvée, laquelle s'étend à travers le maxillaire supérieur et/ou le maxillaire inférieur d'une tête modèle ou d'un préenregistrement de la tête du patient.

5. Procédé selon la revendication 4, **caractérisé en ce que** la surface cible (22) est plane dans une direction perpendiculaire à un plan occlusal du maxillaire supérieur (2) et/ou du maxillaire inférieur (3) ou incurvée dans une direction suivant le tracé des axes dentaires du maxillaire supérieur (2) et/ou du maxillaire inférieur (3).

6. Procédé selon la revendication 4 ou 5, **caractérisé en ce que** l'image composite (29) correspond à ou reconstitue une image panoramique en couches par rayons X conventionnelle.

7. Procédé selon l'une quelconque des revendications 1 à 6, **caractérisé en ce que** la surface cible (22) de l'image composite (29) correspond au ou reconstitue le tracé d'une couche nette d'une image panoramique en couches par rayons X conventionnelle.

8. Procédé selon l'une quelconque des revendications 1 à 7, **caractérisé en ce que** les zones de volume de segment (6, 9, 12, 15, 18) respectives sont disposées de telle sorte qu'une direction de la résolution la plus petite de l'image de segment IRM respective est disposée orthogonalement à la surface cible (22).

9. Procédé selon l'une quelconque des revendications 1 à 8, **caractérisé en ce qu'**avant la création des enregistrements de segment IRM (5, 8, 11, 14, 17), le nombre d'enregistrements de segment IRM (5, 8, 11, 14, 17), la position et/ou l'orientation des enregistrements de segment IRM (5, 8, 11, 14, 17) par rapport au dispositif d'IRM (4) peuvent être définis par un utilisateur, dans lequel au moyen d'un outil virtuel, la position et l'orientation des zones de volume de segment (6, 9, 12, 15, 18) des enregistrements de segment IRM sont définies par rapport au dispositif d'IRM (4).

10. Procédé selon l'une quelconque des revendications 1 à 9, **caractérisé en ce qu'**une tête modèle comportant un maxillaire supérieur modèle et/ou un maxillaire inférieur modèle est représentée schématiquement au moyen d'un dispositif de visualisation (24), dans lequel les zones de volume de segment (6, 9, 12, 15, 18) définies et leur disposition par rapport à la tête modèle et/ou la zone cible (22) définie par rapport à la tête modèle sont représentées à l'aide d'une représentation graphique (28).

11. Procédé selon la revendication 10, **caractérisé en ce que**, au moyen du dispositif de visualisation (24), l'image composite (29) générée et la tête modèle comportant la représentation graphique des zones de volume de segment (5, 8, 11, 14, 17) individuelles et/ou comportant la zone cible (22) définie peuvent être représentées simultanément.

12. Procédé selon l'une quelconque des revendications 1 à 11, **caractérisé en ce que** chaque image de segment IRM (5, 8, 11, 14, 17) comprend une seule image en couches IRM ou comprend un empilement de plusieurs images en couches IRM.

13. Procédé selon l'une quelconque des revendications 1 à 12, **caractérisé en ce que** dans tous les enregistrements de segment IRM (5, 8, 11, 14, 17), la position spatiale de l'objet dentaire (1) par rapport au volume de mesure (21) du dispositif d'IRM (4) reste constante.
